**Europäisches Patentamt**

**European Patent Office**

**Office européen des brevets**

(19)

(11) Numéro de publication : **0 485 278 A1**

(12) ## DEMANDE DE BREVET EUROPEEN

(21) Numéro de dépôt : **91402965.7**

(22) Date de dépôt : **06.11.91**

(51) Int. Cl.⁵ : **A61B 17/30, A61B 17/28**

(30) Priorité : **06.11.90 FR 9013712**

(43) Date de publication de la demande :
**13.05.92 Bulletin 92/20**

(84) Etats contractants désignés :
**AT BE CH DE DK ES GB GR IT LI LU NL**

(71) Demandeur : **ETHICON INC.**
**U.S. Route 22**
**Somerville New Jersey 08876 (US)**

(72) Inventeur : **Bilweis, Joseph**
**10, l'Orée de Marly**
**F-78560 Noisy le Roi (FR)**

(74) Mandataire : **Martin, Jean-Jacques et al**
**Cabinet REGIMBEAU 26, Avenue Kléber**
**F-75116 Paris (FR)**

(54) **Instrument chirurgical endoscopique pour la saisie de tissus.**

(57) La présente invention concerne un instrument chirurgical, notamment pour chirurgie
endoscopique, caractérisé par le fait qu'il
comprend un tube (10) pourvu à son extrémité
proximale (12) d'une ventouse souple (13) et à
son extrémité distale (16) d'une poire (17), tel
que le volume (18) de la poire (17) communique
avec la ventouse (13) par l'intermédiaire du tube
(10) pour permettre par pression/dépression de
la poire (17) de contrôler l'application et l'adhérence de la ventouse (13) sur des tissus ou
organes à déplacer.

FIG.1

EP 0 485 278 A1

La présente invention concerne le domaine des instruments chirurgicaux.

La présente invention vise en particulier le domaine de la chirurgie endoscopique.

Les chirurgiens savent que les instruments jusqu'ici proposés pour saisir et/ou déplacer des tissus et/ou organes, notamment lors d'interventions endoscopiques, ne donnent pas totalement satisfaction.

Ces instruments sont généralement formés de pinces ou équivalents et occasionnent fréquemment des traumatismes sur les tissus ou organes saisis.

La présente invention a pour but d'éliminer les inconvénients des instruments antérieurs connus.

Ce but est atteint selon la présente invention grâce à un instrument chirurgical, notamment pour chirurgie endoscopique, comprenant un tube pourvu à son extrémité proximale d'une ventouse souple et à son extrémité distale d'une poire, tel que le volume interne de la poire communique avec la ventouse par l'intermédiaire du tube, pour permettre par pression/dépression de la poire de contrôler l'application et l'adhérence de la ventouse sur des tissus ou organes à saisir ou déplacer.

D'autres caractéristiques, buts et avantages de la présente invention apparaîtront à la lecture de la description détaillée qui va suivre, et en regard du dessin unique annexé qui représente à titre d'exemple non limitatif, selon une vue en coupe longitudinale axiale, un instrument conforme à la présente invention.

On aperçoit sur la figure annexée un instrument apte à saisir ou déplacer des tissus ou organes d'un être vivant et qui comprend essentiellement un tube 10 muni à son extrémité proximale 12 d'une ventouse 13 et muni à son extrémité distale 16 d'une poire 17.

La ventouse 13 est formée d'une calotte concave munie d'un orifice central 14, de sorte que la ventouse 13 communique avec le volume interne 18 de la poire 17 par l'intermédiaire du canal longitudinal interne 11 du tube 10.

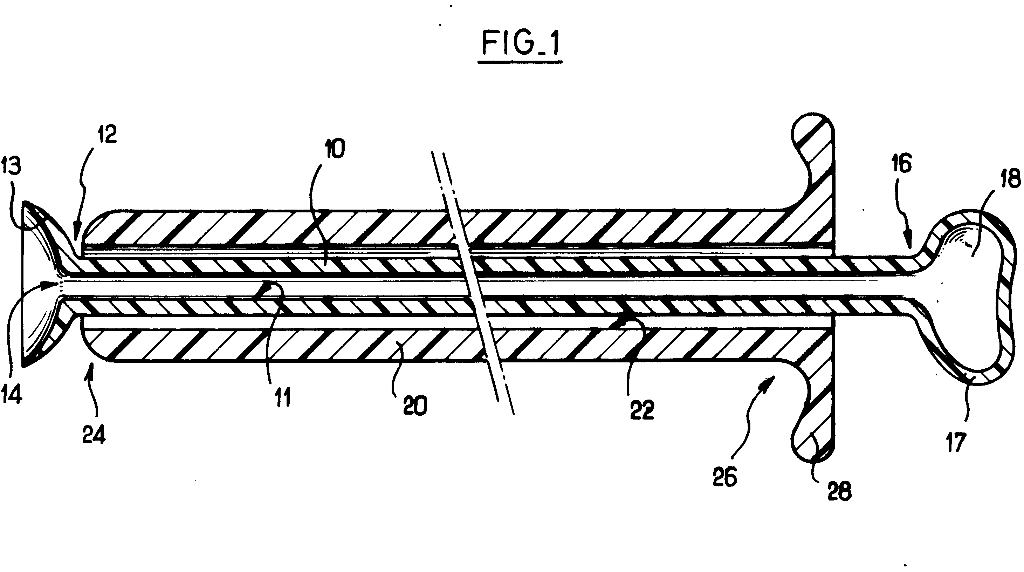

La représentation donnée sur la figure 1 n'est bien entendu que schématique. En particulier les dimensions relatives du diamètre du tube 10, de la ventouse 13 et de la poire 17 représentés schématiquement sur la figure 1 ne sont aucunement limitatives.

De préférence le tube 10, la ventouse 13 et la poire 17 sont réalisés sous forme d'une pièce unique. Cependant, le tube 10, la ventouse 13 et la poire 17 peuvent être réalisés sous forme de pièces initialement séparées puis assemblés à l'aide de toute technique connue de l'homme de l'art.

Le matériau composant la ventouse 13 et la poire 17 doit être un matériau souple élastique pour permettre à ces éléments de recouvrir leur position de repos après sollicitation. De préférence le matériau composant la ventouse 13 et la poire 17 est du caoutchouc.

De façon avantageuse comme cela est représenté schématiquement sur la figure 1, le tube 10 est placé dans la lumière 22 d'une canule 20. Cette canule 20 peut faire l'objet de nombreuses variantes de réalisation.

De préférence l'extrémité proximale 24 de la canule 20 est arrondie pour éviter de blesser les tissus ou organes saisis ainsi que les tissus ou organes environnants ceux qui sont saisis par la ventouse 13. Par ailleurs, de préférence la canule 20 est munie à son extrémité distale 26 de moyens de préhension, tels que par exemple d'une collerette 28.

Comme représenté sur la figure 1, le tube 10 émerge respectivement aux deux extrémités de la canule 20 de sorte que la ventouse 13 soit accessible à l'extrémité proximale de la canule 20 tandis que la poire 17 est accessible à l'extrémité distale de celle-ci.

Le cas échéant le tube 10 peut être placé dans une canule 20 possédant plusieurs lumières longitudinales séparées, les autres lumières étant susceptibles d'être utilisées à des fins connues en soi, telles que par exemple pour l'écoulement de fluide de traitement ou le prélèvement.

Pour saisir un tissu ou organe à l'aide de la ventouse 13, il suffit, après avoir comprimé au moins en partie la poire 17 d'appliquer la ventouse sur le tissu ou organe à saisir et de relacher simultanément la poire 17. Pour libérer ensuite le tissu ou organe ainsi saisi, il suffit de comprimer à nouveau la poire 17.

La saisie de tissu ou organe par aspiration à l'aide de la ventouse 13 permet d'éviter en toute sécurité tout traumatisme au niveau des tissus ou organes saisis.

Bien entendu la présente invention n'est pas limitée au mode de réalisation particulier qui vient d'être décrit mais s'étend à toutes variantes conformes à son esprit.

**Revendications**

1. Instrument chirurgical, notamment pour chirurgie endoscopique, caractérisé par le fait qu'il comprend un tube (10) pourvu à son extrémité proximale (12) d'une ventouse souple (13) et à son extrémité distale (16) d'une poire (17), tel que le volume (18) de la poire (17) communique avec la ventouse (13) par l'intermédiaire du tube (10) pour permettre par pression/dépression de la poire (17) de contrôler l'application et l'adhérence de la ventouse (13) sur des tissus ou organes à déplacer.

2. Instrument chirurgical selon la revendication 1, caractérisé par le fait que le tube (10), la ventouse (13) et la poire (17) sont réalisées sous forme d'une pièce unique.

3. Instrument selon la revendication 1, caractérisé par le fait que l'ensemble formé par le tube (10), la ventouse (13) et la poire (17) est réalisé par assemblage de pièces séparées.

4. Instrument selon l'une des revendications 1 à 3, caractérisé par le fait que la ventouse (13) est réalisée en caoutchouc.

5. Instrument selon l'une des revendications 1 à 4, caractérisé par le fait que la poire (17) est réalisée en caoutchouc.

6. Instrument selon l'une des revendications 1 à 5, caractérisé par le fait que le tube (10) est placé dans une canule (20).

FIG. 1

EP 0 485 278 A1

EP 0 485 278 A1

Office européen
des brevets

**RAPPORT DE RECHERCHE EUROPEENNE**

Numero de la demande

EP  91 40 2965

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | US-A-4 123 098 (SHOUP)<br>* revendication 1; figure 1 *<br>--- | 1 | A61B17/30<br>A61B17/28 |
| A | SU-A-571 246 (MOSCOW LOMONOSOV UN)<br>* abrégé; figure 1 *<br>--- | 1 | |
| A | JP-A-6 118 920 (YASUHIKO)<br>* Resume en anglais *<br>--- | 1 | |
| A | GB-A-1 388 858 (PEARCE)<br>--- | | |
| A | DE-A-3 920 919 (SCHNEIDER)<br><br>----- | | |

| | |
|---|---|
| **DOMAINES TECHNIQUES RECHERCHES (Int. Cl.5 )** |
| A61B<br>A61F |

Le présent rapport a été établi pour toutes les revendications

| Lieu de la recherche | Date d'achèvement de la recherche | Examinateur |
|---|---|---|
| LA HAYE | 14 FEVRIER 1992 | BARTON S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P0402)

5